Europäisches Patentamt

**European Patent Office**

Office européen des brevets

(19)

(11) **EP 1 079 815 B1**

(12) **EUROPÄISCHE PATENTSCHRIFT**

(45) Veröffentlichungstag und Bekanntmachung des Hinweises auf die Patenterteilung:
**20.10.2004 Patentblatt 2004/43**

(51) Int Cl.⁷: **A61K 9/70**

(21) Anmeldenummer: **99926381.7**

(86) Internationale Anmeldenummer:
**PCT/EP1999/003554**

(22) Anmeldetag: **25.05.1999**

(87) Internationale Veröffentlichungsnummer:
**WO 1999/061007 (02.12.1999 Gazette 1999/48)**

(54) **ZEITGESTEUERT FREISETZENDE WIRKSTOFFHALTIGE TRANSDERMALSYSTEME**

TIMED-RELEASE TRANSDERMAL SYSTEMS CONTAINING AN ACTIVE INGREDIENT

SYSTEME TRANSDERMIQUE A LIBERATION CONTROLEE, RENFERMANT UN PRINCIPE ACTIF

(84) Benannte Vertragsstaaten:
**DE ES FR GB IT**

(30) Priorität: **22.05.1998 DE 19823027**
**22.09.1998 DE 19844079**

(43) Veröffentlichungstag der Anmeldung:
**07.03.2001 Patentblatt 2001/10**

(73) Patentinhaber: **Novosis AG**
**83714 Miesbach (DE)**

(72) Erfinder: **FISCHER, Wilfried**
**D-80807 München (DE)**

(74) Vertreter: **Biagosch, Stephan**
**Grafinger Strasse 2**
**81671 München (DE)**

(56) Entgegenhaltungen:
**EP-A- 0 186 019        EP-A- 0 208 395**
**EP-A- 0 227 836        WO-A-89/07959**
**WO-A-95/24172        WO-A-98/00118**
**DE-A- 3 333 444        DE-C- 19 517 145**
**US-A- 5 071 656**

EP 1 079 815 B1

**Beschreibung**

Die vorliegende Erfindung betrifft wirkstoffhaltige Transdermalsysteme mit zeitgesteuerter Freisetzungschanakteristik. Die erfindungsgemäßen Transdermalsysteme können, einmal auf die Haut aufgebracht, ihren Wirkstoff in zwei oder mehreren aufeinanderfolgenden Stufen mit unterschiedlichen Geschwindigkeiten abgeben. Dadurch können dem Organismus, anders als bei Systemen des Stands der Technik, zu unterschiedlichen Zeiten während des Applikationsintervalls unterschiedliche Wirkstoffmengen verabreicht werden.

Die transdermale Gabe von Medikamenten mittels transdermaler therapeutischer Systeme ("Pflaster") ist heute Stand der medizinischen Anwendungen. Es werden erfolgreich solche Wirkstoffe mit Pflastern verabreicht, bei denen für die Wirksamkeit des Arzneimittels ein gleichförmiger Blutspiegelverlauf erwünscht ist. Zu dieser Kategorie gehören Wirkstoffe wie Estradiol, Clonidin, Fentanyl, Scopolamin, Flurbiprofen, Diclofenac etc. Die pharmakologische Wirkung dieser Wirkstoffe bleibt auch dann erhalten, wenn sie über längere Zeit, z.B. einige Tage, verabreicht werden. Diese Wirkstoffe können auf einfache Art und Weise in sog. Matrix-Pflaster eingebettet werden, die dem Fachmann hinreichend bekannt sind: Matrix-Pflaster zeichnen sich vor anderen Pflastersystemen durch eine rein diffusionsgesteuerte Wirkstofffreisetzung aus, die nach der von Higuchi aufgestellten Diffusionsgleichung, 2. Wurzelgesetz (Gl. 1), beschrieben werden kann.

$$dQ_t = \frac{1}{2}\, k_1\, A\, t^{\frac{1}{2}} \qquad \text{(Gl. 1)}$$

Q   Arzneistoffmasse
$k_1$   Liberationskonstante
A   Fläche
t   Zeit

Für homogene Matrices ist $k_1$ entsprechend Gl. 2 einzusetzen

$$k_1 = \left[ D\, c_s \left( \frac{2M_0}{V} - c_s \right) \right]^{\frac{1}{2}}$$

Gl. 2

D   Diffusionskoeffizient in Matrix
$c_s$   Sättigungskonzentration Arzneistoff in Matrix
$M_0$   Wirkstoffmenge in Matrix, t=0
V   Matrixvolumen

Dieser Gleichung (1), der Grundgleichung für alle matrixkontrollierten Arzneiformen, ist zu entnehmen, daß die Wirkstoffabgabe zur Quadratwurzel der Zeit umgekehrt proportional ist, d.h. die freigesetzte Menge mit der Zeit stetig abnimmt. Für die resultierenden Blutspiegel ergibt sich hieraus prinzipiell ein stärkerer Anstieg direkt nach der Applikation und ein stetiger Abfall über den gesamten Applikationszeitraum.

Ein erneuter Anstieg der Blutspiegel ist nur nach Auswechseln des Pflasters durch ein unverbrauchtes zu erreichen.

Eine komplizierter aufgebaute Variante von Transdermalsystemen ist die der Membranpflaster (z.B. DE 2 135 533, Alza Corp.). Sie sind mit dem Ziel einer zeitkonstanten Wirkstoffabgabe konstruiert worden. Die Freisetzung von Wirkstoffen wird in Gl. 3 (Permeation aus Reservoir durch Membranen) beschrieben.

$$\frac{dQ}{dt} = \frac{K_{m/r}\, K_{a/m}\, D_a\, q}{K_{m/r}\, D_m\, h_a + K_{a/m}\, D_a\, h_m} \qquad \text{Gl. 3}$$

dQ/dt   Liberationsgeschwindigkeit
$K_{m/r}$   Verteilungskoeffizient Membran/Reservoir
$K_{a/m}$   Verteilungskoeffizient Haftschicht/Membran
$D_m$   Diffusionskoeffizient in Membran
$D_a$   Diffusionskoeffizient in Haftschicht
q   Wirkstoffmenge
$h_m$   Dicke der Membran
$h_a$   Dicke der Haftschicht

Auch nach dieser Gleichung ist die Wirkstoffabgabe stetig und bei langen Zeiten, q wird kleiner, monoton abnehmend. Im Falle des - unerwünschten - Risses der Membran kann es zu einem Dose dumping kommen, einem Freisetzungsimpuls, bei dem aber keinerlei Kontrolle über die Freisetzung ausgeübt werden kann.

Neben Wirkstoffen, die eine kontinuierliche Verabreichung während eines längeren Zeitraums ermöglichen, sind aber auch Wirkstoffe bekannt, die während der Applikationsperiode variable Blutkonzentrationen erfordern. Die bekanntesten Beispiele dafür, die auch in Transdermalsystemen angeboten werden, sind Nitroglycerin, Nicotin und Testosteron. Nitroglycerin führt bei kontinuierlicher Zufuhr nach einigen Stunden zu einem durch Toleranzentwicklung eintretenden Wirksamkeitsverlust. Während der ersten Jahre der Vermarktung der Produkte trug man diesem Umstand nicht genügend Rechnung, so daß diese Art der Therapie mit Nitroglycerin in Fachkreisen nur eine geringe Akzeptanz fand. Entsprechend neueren Therapieerfahrungen werden heute die Pflaster nur ca. 10 - 12 Stunden appliziert, dann zur Schaffung einer Regenerierungsphase abgenommen, so daß abends kein Schutz durch Nitrate gegeben ist. Gerade in den frühen Morgenstunden treten jedoch gehäuft Anfälle von Angina pectoris auf. Bei dieser Applikationsart ist dann kein Schutz gewährleistet, es sei denn, die Patienten applizieren die Pflaster vor dem Aufstehen.

Die auf dem Markt befindlichen Pflaster für Nitroglycerin berücksichtigen diesen tageszeitlich variierenden Wirkstoffbedarf nicht.

Die Applikation nikotinhaltiger Pflaster soll zur Raucherentwöhnung beitragen. Die stimulierende Wirkung des Nikotins entfaltet sich bei dem plötzlichen Anstieg der Nikotinkonzentration im Blut beim Rauchen einer Zigarette o.ä. Die im Handel befindlichen Matrix-Nikotinpflaster können aber nur einen zeitkonstanten Blutspiegel erzeugen, der nicht den individuellen Nikotinbedarf des Rauchers decken kann.

Testosteron wird dem Organismus physiologischerweise in einem circadianen Rhythmus systemisch zur Verfügung gestellt. In den Morgenstunden um ca. 8:00 Uhr ist seine Blutkonzentration am höchsten, sinkt dann gegen Mittag auf ein relatives Minimum, um nachmittags wieder leicht anzusteigen. In der Nacht sind die Blutspiegel am geringsten. In der WO A 9210231 und in der DE 195 17 145 werden transdermale Systeme beschrieben, die morgens appliziert relativ rasch zu einem Blutspiegelpeak führen, der gegen Nachmittag wieder absinkt. Der zweite Peak am Nachmittag kann mit diesen Systemen nicht erzielt werden. Bei dem zitierten Stand der Technik handelt es sich um Reservoirsysteme, bei denen die Wirkstofffreisetzung direkt nach der Applikation beginnt. Das Wirkstoffreservoir besteht im wesentlichen aus alkoholischen Wirkstofflösungen, die durch poröse Membranen in die Haut diffundieren. Dabei steht die mit Wirkstofflösung gesättigte Membran direkt mit der Haut in Kontakt. Derartige Systeme sind schwierig herzustellen und sehr teuer.

Die Aufgabe der vorliegenden Erfindung besteht nun darin, ein auf Standardmaschinen herzustellendes, preiswertes Matrix-Transdermalsystem zur Verfügung zu stellen, das den Anforderungen an den zeitabhängigen Wirkstoffbedarf des Körpers gerecht wird.

Insbesondere soll es das System ermöglichen, dem menschlichen oder tierischen Körper in variabler Weise Wirkstoffe zur Verfügung zu stellen.

Erfindungsgemäß wird daher ein Transdermalsystem gemäß Anspruch 1 bereitgestellt.

Das erfindungsgemäße System weist den Vorteil auf, daß die hydrophilen Polymere der Schicht (3) durch Hautfeuchtigkeit anoder aufgelöst werden können, wodurch es zum vollständigen Zusammenbruch der Schicht (3) kommen kann. Dann werden die Wirkstoffe stoßartig freigesetzt und an die Haut des Patienten abgegeben.

Durch Einstellung der Beladung, Dicke, Zusammensetzung, Porengröße, Wasserdurchlässigkeit etc. der Schicht (4) und gegebenenfalls auch der Schicht (3) kann der Zeitpunkt genau eingestellt werden, an dem es zu der Freisetzung des Wirkstoffs oder des Wirkstoffgemisches kommt.

Enthält auch die Schicht (4) einen oder mehrere Wirkstoffe, so wird beginnend mit dem Aufbringen des Systems auf die Haut langsam Wirkstoff an die Haut abgegeben. Diese Phase kann bis zu dem Zeitpunkt dauern, an dem die stoßartige Wirkstofffreisetzung der Schicht (3) einsetzt.

In einer bevorzugten Ausführungsform sind die Polymere der Schicht (3) in Wasser löslich.

In einer bevorzugten Ausführungsform weist die Schicht (3) Perforationen (Löcher) auf, durch die die Haftkleberschicht (4) mit der Abdeckschicht (1) in Kontakt treten kann. Dadurch wird die Haftung der Abdeckschicht (1) auf der Polymerschicht (3) verstärkt. Der Durchmesser der Löcher kann z.B. 0,1 bis 5 mm, vorzugsweise 0,5 bis 2 mm betragen.

Das erfindungsgemäße Transdermalsystem kann eine zwischen der wirkstoffhaltigen Polymerschicht (3) und der Abdeckschicht (1) angeordnete Haftkleberschicht (2) aufweisen, die gegebenenfalls wirkstoffhaltig ist.

Dieses System ist dann besonders vorteilhaft, wenn es zum Zusammenbruch der Polymerschicht (3) gekommen ist, da das Pflaster weiter seine Stabilität beibehält.

Insbesondere, wenn die Schicht (3) perforiert ist und Verankerungen durch die Perforation von Schicht (2) zu Schicht (4) führen, wird ein erheblicher Stabilitätsgewinn erzielt.

Enthält Schicht (2) einen oder mehrere Wirkstoffe, so kann nach der stoßartigen Freisetzung durch Schicht (3) weiter langsam Wirkstoff an die Haut abgegeben werden.

Die Abdeckschicht (1) umfaßt erfindungsgemäß an sich übliche Materialien wie ein oder mehrere wasserdampfundurchlässige(s) Material(ien), wie Polyterephthalsäureester oder Polypropylen, oder ein oder mehrere wasserdampfdurchlässige(s) Material(ien), wie Polyurethan, oder ein oder mehrere Gewebe oder Vliese.

In einer bevorzugten Ausführungsform sind die Haftkleberschicht (4) und gegebenenfalls die Haftkleberschicht (2) unabhängig voneinander druckempfindliche Haftkleberschichten.

Erfindungsgemäß sind der oder die Wirkstoff(e) in der Polymerschicht (3) vorzugsweise nicht mit Wasser mischbar.

Als hydrophile Polymere kommen z.B. Gelatine oder Celluloseester oder -ether oder Derivate davon in Betracht.

Die erfindungsgemäß eingesetzten Wirkstoffe können in den jeweiligen Schichten als immobilisierte Wirkstofflösung(en) oder -dispersion(en) vorliegen.

Die Schutzschicht (5) kann eine an sich übliche, wiederablösbare Schutzschicht sein und eine silikonisierte Folie oder ein Silikonpapier umfassen.

Das erfindungsgemäße Transdermalsystem kann so aufgebaut sein, daß

     a) die Schichten (2) und (4) wirkstoffrei sind, oder
     b) die Schicht (2) wirkstoffhaltig und die Schicht (4) wirkstoffrei ist, oder
     c) die Schicht (2) wirkstoffrei und die Schicht (4) wirkstoffhaltig ist, oder

    d) die Schichten (2) und (4) wirkstoffhaltig sind,

wobei die Dicke der Schicht (4) 10 bis 300 μm, vorzugsweise 30 bis 100 μm und die Dicke der Schicht (2) 1 bis 300 μm, vorzugsweise 3 bis 100 μm betragen kann.

Als Wirkstoffe kommen z.B. Testosteron, Nitroglycerin, Nicotin oder Gemische davon in Betracht.

Somit kann das erfindungsgemäße Transdermalsystem z.B. zur Behandlung von Angina pectoris, zum Nicotinentzug oder bei Testosteronmangelerscheinungen eingesetzt werden.

Ein erfindungsgemäßes Verfahren zur Herstellung eines erfindungsgemäßen Transdermalsystems ist dadurch gekennzeichnet, daß eine Haftschicht (4) auf eine Schutzschicht (5) aufgebracht wird, auf die Haftschicht (4) eine wirkstoffhaltige, gegebenenfalls Perforationen aufweisende hydrophile Polymerschicht (3) aufgebracht wird, auf die Polymerschicht (3) gegebenenfalls eine weitere Haftschicht (2) aufgebracht und auf die oberste Schicht eine Abdeckschicht (1) aufgebracht wird.

Das erfindungsgemäße Transdermalsystem besteht also aus einem mehrschichtigen Laminat, umfassend eine Abdeckschicht (backing, Schicht (1)), die wasserdampfundurchlässig, wie Polyterephthalsäureester oder Polypropylen, oder wasserdampfdurchlässig sein kann, wie Polyurethan, oder ein Gewebe oder ein Vlies. Auf diese Abdeckschicht wird gegebenenfalls eine druckempfindliche Haftkleberschicht (Verankerung, Schicht (2)), druckempfindliche Haftkleber sind jedem Fachmann hinreichend bekannt, entweder aus Lösung, Dispersion oder lösemittel- oder dispersionsmittelfrei, aufgebracht. Diese Schicht kann wirkstoffhaltig oder wirkstofffrei sein, vorzugsweise ist sie wirkstofffrei. Auf diese Klebstoffschicht wird eine Schicht (Schicht (3)), umfassend oder bestehend aus einer Polymerschicht und einer in der Polymerschicht immobilisierten Wirkstofflösung oder -dispersion, aufgebracht. Das Polymer ist vorzugsweise ein wasserlösliches Polymer, wie Gelatine oder Celluloseester oder -ether. In das wasserlösliche Polymer (Polymerschicht (3)) wird z.B. eine nicht wassermischbare Wirkstofflösung oder -dispersion eingebracht, die das alleinige oder einen Teil des gesamten Wirkstoffreservoirs bilden kann. Auf die Polymerschicht wird eine weitere Schicht (Schicht (4)) 'eines druckempfindlichen Haftklebers, die zum Fixieren des Systems auf der Haut vorgesehen ist, aufgebracht. Diese Schicht kann wirkstoffhaltig oder wirkstoffrei sein. Sie umfaßt oder besteht z.B. aus den dem Fachmann bekannten druckempfindlichen Haftklebern, die wiederum aus Lösung, Dispersion oder lösemittel- oder dispersionsmittelfrei aufgetragen werden können. Es können Verankerungen durch Löcher in der Polymerschicht (3) hindurchgezogen werden, um Schicht (2) mit Schicht (4) zu verbinden. Auf die letztgenannte Schicht wird eine wiederablösbare Schutzschicht (Schicht (5)), in der Regel eine silikonisierte Folie oder ein Silikonpapier, aufgebracht, die vor der Applikation des Systems auf die Haut entfernt wird.

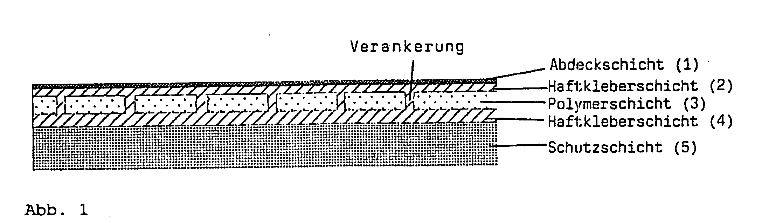

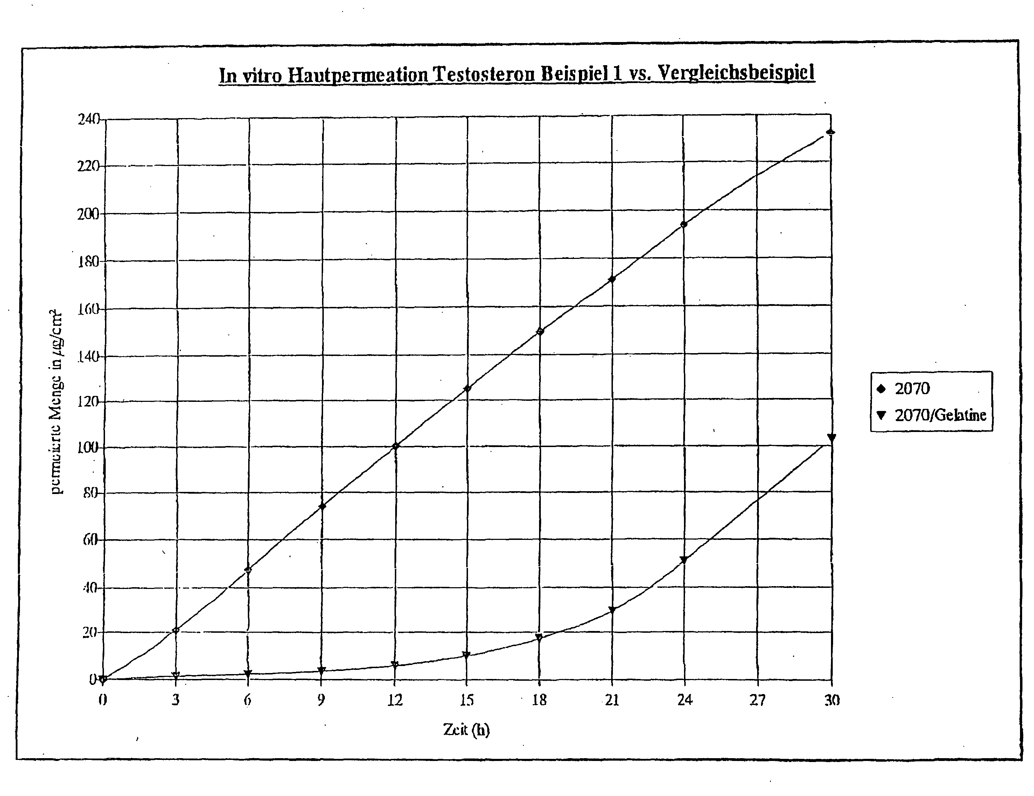

Zur Erläuterung der Funktionsweise des erfindungsgemäßen Systems können 4 Fälle unterschieden werden (s. Abb. 1):

i. Schicht (2) und Schicht (4) sind wirkstoffrei

Da die Polymerwirkstoffschicht (Schicht (3)) aus einem oder mehreren wasserlöslichen Polymer(en) bestehen oder diese(s) umfassen kann, muß die Hautfeuchtigkeit in diese Schicht eindringen, sie an- oder auflösen, um den in ihr z.B. als Emulsionströpfchen immobilisierten Wirkstoff herausdiffundieren zu lassen. Der, z.B. je nach Beladungsgrad der Schicht (3), mehr oder weniger schnelle Zusammenbruch der Schicht führt zum stoßartigen Freiwerden einer großen Menge des Wirkstoffs, der schnell durch die Schicht (4) in die Haut eindringen kann.

Ein weiterer Vorteil des erfindungsgemäßen Systems gegenüber den herkömmlichen Matrixsystemen besteht darin, daß nach Aufkleben des Systems auf die Haut über eine voreinzustellende Zeit keine Wirkstoffabgabe erfolgt, dann aber eine stoßartige Wirkstoffabgabe erfolgt. Die Voreinstellung der Zeit kann z.B. durch die Dicke der Schicht (4), deren Zusammensetzung, Porengröße, Wasserdurchlässigkeit etc. eingestellt werden.

ii. Schicht (2) ist wirkstoffhaltig, Schicht (4) ist wirkstoffrei

Nach dem unter i beschriebenen Erschöpfen der Polymerschicht (3) kann nun aus der Schicht (2) weiterer Wirkstoff nachdiffundieren und eine zweite kontinuierliche Freisetzungsperiode beginnen. Diese Ausbildung ist vorteilhaft für Wirkstoffe wie Testosteron einsetzbar, bei denen nach einem anfänglich schnellen Freisetzungsanteil ein langsamerer kontinuierlich freisetzender Dosisteil benötigt wird.

iii. Schicht (2) ist wirkstofffrei, Schicht (4) ist Wirkstoffhaltig

Bei dieser Ausbildung des erfindungsgemäßen Systems beginnt direkt nach der Applikation die kontinuierliche Wirkstofffreisetzung aus der Schicht (4). Nach einer durch die Dicke und Zusammensetzung etc. der Schicht (4) bestimmten Verzögerungszeit beginnt die unter i beschriebene, schnelle Freisetzung des Wirkstoffanteils der Schicht (3). Diese Systeme können vorteilhaft dort eingesetzt werden, wo z.B. in Zeiten körperlicher Ruhe keine oder nur geringe Krankheitssymptome auftreten, aber zu Zeiten von beginnenden Aktivitäten diese Symptome deutlich stärker werden. Es ist z. B. für organische Nitrate bekannt, die bei der Behandlung von koronaren Herzkrankheiten verwendet werden, daß sie in den Morgenstunden, zur Zeit der häufigsten Herzanfälle, ihre höchste Wirksamkeit entfalten. In Phasen der relativen körperlichen Ruhe ist der Bedarf an Nitrat dagegen niedriger. Ein derartiges System könnte also abends appliziert werden, gibt dann nur relativ wenig Wirkstoff während der Ruhezeit ab, um morgens, nach der entsprechend eingestellten Verzögerungszeit, mehr Wirkstoff an den Körper abzugeben.

iv. Schicht (2) und Schicht (4) sind wirkstoffhaltig

Bei dieser Ausbildung des erfindungsgemäßen Transdermalsystems beginnt die kontinuierliche Freisetzung des Wirkstoffes nach der Applikation aus der Schicht (4), erreicht nach der Verzögerungszeit ihren Maximalwert, um nach Erschöpfen der Polymerschicht (3) wieder langsam und kontinuierlich aus der Schicht (2) fortgesetzt zu werden.

Herstellungsverfahren

Die druckempfindliche Haftschicht (Schicht (4)) wird - mit oder ohne darin gelöstem oder dispergiertem Wirkstoff - auf die silikonisierte Schutzschicht (Schicht (5)) aufgetragen. Dabei kann die Schicht aus Lösung in organischen Lösemitteln wie Ethylacetat, Hexan o.a., aus Lösung oder Dispersion in wäßrigen Lösemitteln oder auch lösemittelfrei mittels Schmelzbeschichtung oder durch in situ Polymerisation aufgetragen werden. Die Stärke dieser Schicht kann 10 bis 300 μm, vorzugsweise 30 bis 100 μm betragen. Der (die) darin enthaltene(n) Wirkstoff(e) kann (können) echt gelöst oder mikrodispers vorliegen. Es sind Kombinationen mehrerer Wirkstoffe einsetzbar.

In einem separaten Arbeitsgang wird die wirkstoffhaltige Polymerschicht (Schicht (3)) hergestellt, indem z.B. eine Lösung oder Dispersion eines oder mehrerer Wirkstoffe in einem nicht- oder schwerflüchtigen Lösemittel in eine wäßrige Lösung oder Schmelze eines hydrophilen Polymers einemulgiert wird. Die Emulsion wird auf einer Intermediärfolie ausgestrichen. Beim Erstarren der Emulsion bilden sich z.B. flüssigkeitsgefüllte Poren in der Polymerschicht. Diese Schicht wird nachgetrocknet und ergibt einen Film, in dem die Wirkstofflösung/Dispersion in immobilisierten Tropfen vorliegt. Dann kann die Polymerschicht mittels Stanzung mit Löchern mit Durchmessern von 0,1 bis 5 mm, vorzugsweise 0,5 bis 2 mm versehen werden. Danach kann die gelochte Polymerschicht unter Entfernen der Schutzfolie auf die Schicht (4) aufkaschiert werden.

Dann wird in einem weiteren Arbeitsgang die Schicht (2) - mit oder ohne darin gelöstem oder dispergiertem Wirkstoff - auf die Abdeckschicht (Schicht (1)) aufgetragen. Dabei kann die Haftschicht aus Lösung in organischen Lösemitteln wie Ethylacetat, Hexan o.ä., aus Dispersion in wäßrigen Lösemitteln oder auch lösemittelfrei mittels Schmelzbeschichtung oder durch in situ Polymerisation aufgetragen werden. Die Stärke dieser Schicht kann 1 bis 300 μm, vorzugsweise 3 bis 100

μm betragen. Der darin enthaltene Wirkstoff kann echt gelöst oder mikrodispers vorliegen. Es sind Kombinationen mehrerer Wirkstoffe herstellbar. Nach Aushärten dieser Schicht wird sie auf die Polymerschicht (3) aufkaschiert. Dabei kann sich die Haftschicht durch die optional vorhandenen Stanzlöcher der Schicht (3) mit der Haftschicht (4) verbinden. Dadurch ist ein stabiler Verbund des Systems auch dann gewährleistet, wenn sich die Polymerschicht (3) während der Applikation des Systems vollständig auflösen sollte.

Nach Ausstanzen der entsprechenden Kontur aus dem Laminat ist das Transdermalsystem fertiggestellt.

Weiterhin betrifft die Erfindung ein Transdermalsystem, umfassend:

     a) eine wirkstoffundurchdringliche Abdeckschicht: (11),
     b) eine hydrophile und leicht wasserlösliche Membran,
     c) eine auf die Membran aufgeklebte und gegebenenfalls wirkstoffhaltige Haftkleberschicht (14) und
     d) eine ablösbare Schutzschicht (15),

-    wobei die Abdeckschicht (11) und die Membran einen Hohlraum miteinander bilden,
-    in den der Wirkstoff in einem flüssigen Medium eingebracht ist, wobei
-    Abdeckschicht (11) und Membran gegenüber dem wirkstoffhaltigen flüssigen Medium inert sind.

Das weitere erfindungsgemäße Transdermalsystem kann durch eine Membran aus Gelatine, Agar, Stärke oder einem synthetischen hydrophilen und leicht wasserlöslichen Polymermaterial gekennzeichnet sein.

Ferner kann das weitere erfindungsgemäße Transdermalsystem dadurch gekennzeichnet sein, daß die Abdeckschicht (11) ringförmig mit der Membran oder direkt mit der Haftkleberschicht (14) verbunden ist.

Ferner kann das weitere erfindungsgemäße Transdermalsystem dadurchgekennzeichnet sein, daß der Wirkstoff in Form einer Lösung in einem Lösungsmittel vorliegt, insbesondere in einem natürlichen oder synthetischen Öl, vorzugsweise Terpentinöl, Silikonöl oder Neutralöl, oder in einem flüchtigen organischen Lösungsmittel, vorzugsweise Heptan, oder in einem ihrer Gemische.

Ein erfindungsgemäßes Transdermalsystem kann dadurch gekennzeichnet sein, daß die Abdeckschicht (1, 11) ein oder mehrere wasserdampfundurchlässige(s) Material(ien), insbesondere Polyester, vorzugsweise Polyterephthalsäureester, oder Polypropylen oder Polyethylen, oder ein oder mehrere wasserdampfundurchlässige(s) Material(ien), insbesondere Polyurethan, oder ein oder mehrere Gewebe oder Vliese umfaßt.

Ferner kann ein erfindungsgemäßes Transdermalsystem dadurch gekennzeichnet sein, daß die Haftkleberschicht (4, 14) und/oder die Haftkleberschicht (2) unabhängig voneinander druckempfindliche Haftkleberschichten sind.

Ferner kann ein erfindungsgemäßes Transdermalsystem dadurch gekennzeichnet sein, daß die Haftkleberschicht (4, 14) und/oder die Haftkleberschicht (2) ein Netz, Vlies oder Gewebe enthalten, wobei deren Faden- oder Faserstärke vorzugsweise kleiner als die Haftkleberschichtdicke ist.

Ferner kann ein erfindungsgemäßes Transdermalsystem dadurch gekennzeichnet sein, daß die Schutzschicht (5, 15) wiederablösbar ist und insbesondere eine silikonisierte Kunststoffolie oder ein Silikonpapier ist.

Schließlich kann ein erfindungsgemäßes Transdermalsystem dadurch gekennzeichnet sein, daß der Wirkstoff Testosteron, Nitroglycerin oder Gemische davon umfaßt.

Schließlich betrifft die Erfindung die Verwendung eines Transdermalsystems gemäß der Erfindung zur Behandlung von Angina pectoris, zum Nicotinentzug oder bei Testosteronmangelerscheinungen.

**Beispiele**

**1. Testosteronhaltiges Reservoir Transdermalsystem**

Das Beispiel beschreibt ein Reservoirsystem in dem Testosteron in einem terpenhaltigen Pflanzenöl gelöst ist. In der Abbildung 1 ist die in vitro Hautpermeationscharakteristik dieses Systems zusammen mit dem Vergleichsbeispiel dargestellt. Das Vergleichsbeispiel enthält kein die Wirkstoffpermeation zeitlich verzögerndes Element und stellt die Permeation des Testosteron durch eine reine Klebstoffmembrane dar. Die Testpräparate wurden auf die isolierte Haut von Nacktmäusen geklebt und die Wirkstoffdiffusion in bekannten Franz-Zellen untersucht. Es wird deutlich, daß nach einer Lagtime von ca. 15 Stunden die Wirkstofffreisetzung nahezu spontan einsetzt und eine Permeationsgeschwindigkeit wie die der Vergleichspräparation aufweist. Weitere Versuche ergaben, daß die Länge der Lagtime von der Dicke und Beschaffenheit der Lagtimeschicht abhängt.

1.1. Herstellung der Klebstoffschicht
    Ein handelsüblicher druckempfindlicher Haftkleber (Duro-Tak 2070) wird mittels einer geeigneten Beschichtungseinrichtung (z.B. reverse roll coater) auf eine silikonisierte Trägerfolie (z.B. Polyterephthalsäureester) derart aufgetragen, daß ein Flächengewicht des getrockneten Klebstoffes von 30 g/m$^2$ resultiert.

1.2. Herstellung der Lagtimeschicht
    10 g Gelatine, 0,2 g Polysorbat 80, 4,02 g Glycerin und 25,8 g Wasser werden auf ca 80°C erhitzt. Die auf 50°C abgekühlte Gelatinelösung wird mittels einer geeigneten Beschichtungseinrichtung (z.B. reverse roll coater) auf eine Trägerfolie (z.B. Polyterephthalsäureester) derart aufgetragen, daß ein Flächengewicht der getrockneten Gelatine von 45 g/m$^2$ resultiert.

1.3. Herstellung der Wirkstofflösung
    Testosteron wird in einem terpenhaltigen Pflanzenöl bis zur Sättigung gelöst (Konzentration: 8,6%)

1.4. Herstellung des Transdermalsystems
    Aus der Lagtimeschicht aus 1.2 werden kreisförmige Abschnitte mit einem Durchmesser von 1,5 cm ausgestanzt. Die Abschnitte werden auf die nichtabgedeckte Seite der Klebstoffschicht aus 1.1 aufgepreßt. Aus einer Polyterephthalsäureesterfolie einer Stärke von 15 µm (Deckfolie) werden kreisförmige Abschnitte mit einem Durchmesser von 2 cm ausgeschnitten. Diese Folienabschnitte werden in einer geeigneten Montagemaschine zentrisch auf die Gelatinefolienstücke aufgebracht und mit der darunterliegenden Klebeschicht verklebt wobei gleichzeitig mittels einer Füllnadel 0,5 ml der Wirkstofflösung in den entstehenden Hohlraum zwischen Gelatinefolie und Deckfolie eingebracht wird. Nach Herausziehen der Füllnadel wird die Füllöffnung verschlossen und die Transdermalsysteme mit einem Durchmesser von 2 cm ausgestanzt.

**2. Testosteronhaltiges Matrix Transdermalsystem**

Das Beispiel beschreibt ein Matrixsystem, in dem eine ölige Testosteronlösung in einem hydrophilen Polymer immobilisiert ist.

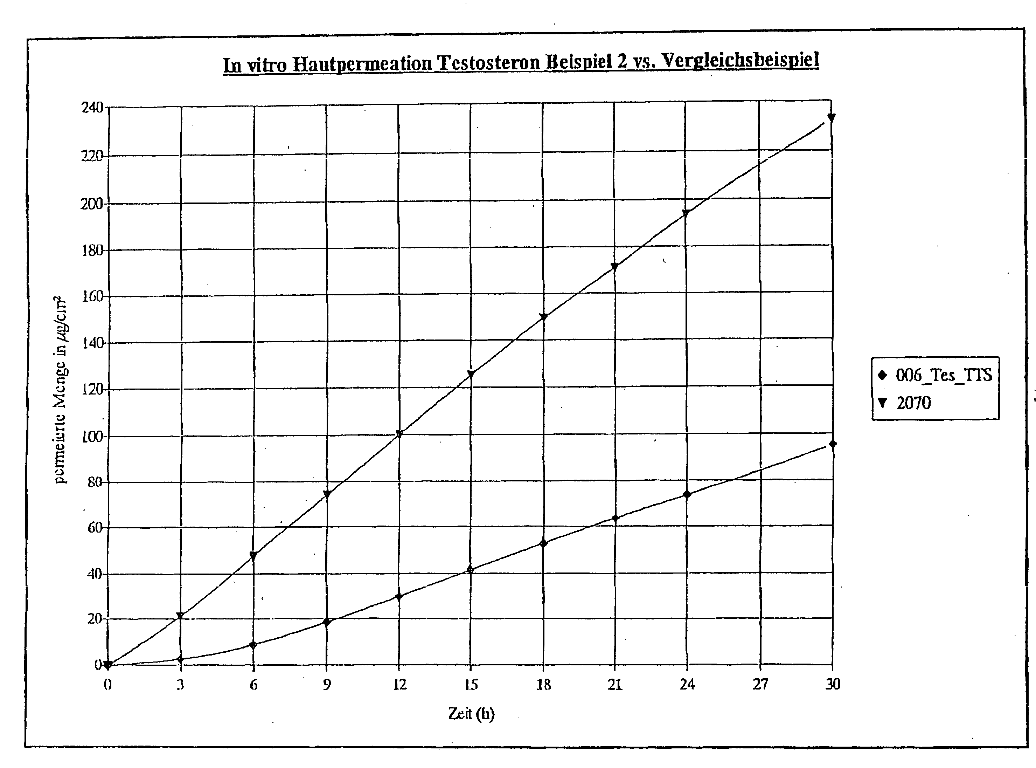

Abbildung 2 zeigt die, unter den Bedingungen wie in 1. beschrieben, in vitro Hautpermeation eines Matrixsystems zusammen mit dem Vergleichsbeispiel. Es ist deutlich eine Lagtime von ca. 3 Stunden zu erkennen.

2.1. Herstellung der Klebstoffschicht wie in 1.1 beschrieben

2.2. Herstellung der Abdeckfolie mit Verankerungsschicht
    Ein handelsüblicher druckempfindlicher Haftkleber (Duro-Tak 2070) wird mittels einer geeigneten Beschichtungseinrichtung (z.B. reverse roll coater) auf eine Trägerfolie (z.B. Polyterephthalsäureester) derart aufgetragen, daß ein Flächengewicht des getrockneten Klebstoffes von 3 g/m$^2$ resultiert.

2.3. Herstellung der Wirkstofflösung wie in 1.3 beschrieben

2.4. Herstellung der wirkstoffhaltigen hydrophilen Polymerschicht 10 g Gelatine, 2,32 g Glycerin, 0,51 g Polysorbat 80 und 26 g Wasser werden bei ca 60°C zu einer homogenen Lösung verarbeitet. 8 g der Wirkstofflösung aus 2.2 werden bei 75°C in die Gelatinelösung einemulgiert. Die Emulsion wird mittels einer geeigneten Beschichtungseinrichtung (z.B. reverse roll coater) auf eine Trägerfolie (z.B. Polyterephthalsäureester) derart aufgetragen, daß ein Flächengewicht der getrockneten Gelatine mit der inkorporierten Wirkstofflösung von 32 g/m$^2$ resultiert.

2.5. Herstellung der Transdermalsysteme

Auf die nicht abgedeckte Seite der Klebstoffschicht aus 2.1 wird die wirkstoffhaltige Gelatineschicht aufkaschiert. Die Klebstoffschicht der Abdeckfolie wiederum wird auf die Gelatineschicht aufkaschiert. Das nun 5-schichtige Laminat, bestehend aus Abdeckfolie, Verankerungsschicht, wirkstoffhaltiger Gelatineschicht, Klebstoffschicht und silikonisierter Polyesterfolie wird in einer geeigneten Stanz- oder Schneidvorrichtung zu Transdermalsystemen mit einem Durchmesser von z. B. 2 cm verarbeitet. Nach Entfernen der silikonisierten Polyesterfolie kann das System mit der druckempfindlichen Haftschicht auf die Haut aufgeklebt werden.

3. Glycerintrinitrathaltiges Matrix Transdermalsystem

Das Beispiel beschreibt ein Matrixsystem bei dem eine ölige Glycerintrinitratlösung in eine Gelatineschicht inkorporiert und mit einer glycerintrinitrathaltigen Klebstoffschicht kombiniert wird.

3.1. Herstellung der glycerintrinitrathaltigen Klebstoffschicht.
Glycerinitrinitrat wird in einer Lösung von Duro-Tak 2052 in einer Menge gelöst, daß eine 10%ige Konzentration in Bezug auf den Feststoffgehalt ergibt. Die Lösung wird mittels einer geeigneten Beschichtungseinrichtung (z.B. reverse roll coater) auf eine Trägerfolie (z.B. silikonisierte Polyterephthalsäureseesterfolie) derart aufgetragen, daß ein Flächengewicht des getrockneten Klebstoffes von 50g/m$^2$ resultiert.

3.2. Herstellung der glycerintrinitrathaltigen Gelatine-schicht
10g Gelatine, 2,32g Glycerin, 0,51g Polysorbat 80 und 26g Wasser werden bei ca. 60°Czu einer homogenen Lösung verarbeitet. 8g einer handelsüblichen 10%igen Lösung von Glycerintrinitrat in Neutralöl werden bei 75°C in die Gelatinelösung einemulgiert. Die Emulsion wird mittels einer geeigneten Beschichtungseinrichtung (z.B. reverse roll coater) auf eine Trägerfolie (z.B. Polyterephthalsäureester) derart aufgetragen, daß ein Flächengewicht der getrockneten Gelatine mit der inkorporierten Wirkstofflösung von 40g/m$^2$ resultiert.

3.3. Herstellung der Deckfolie mit Verankerungsschicht, wie 2.2.

3.4. Herstellung der Transdermalsysteme
Die Herstellung erfolgt wie unter 2.5 beschrieben.

**4. Vergleichsbeispiel**

Die Herstellung des Transdermalsystems erfolgt analog Beispiel 1 wobei die Lagtimeschicht fortgelassen wird.

**Patentansprüche**

1. Transdermalsystem, umfassend

(a) eine Abdeckschicht (1, 11),
(b) ein wasserlösliches Material, das durch Hautfeuchtigkeit auflösbar ist,
(c) eine gegebenenfalls wirkstoffhaltige Haftkleberschicht (4, 14) und
(d) eine davon ablösbare Schutzschicht, wobei das Transdermalsystem **gekennzeichnet ist durch** eine wirkstoffhaltige Polymerschicht (3), in der der Wirkstoff als nicht wassermischbare Wirkstofflösung oder als nicht wassermischbare Wirkstoffdispersion in einem wasserlöslichen Polymeren vorliegt.

2. Transdermalsystem nach Anspruch 1, *gekennzeichnet* **durch** eine zwischen der wirkstoffhaltigen Polymerschicht (3) und der Abdeckschicht (1) angeordnete Haftkleberschicht (2), die gegebenenfalls wirkstoffhaltig ist.

3. Transdermalsystem nach einem der vorstehenden Ansprüche, **dadurch** *gekennzeichnet,* **daß** der oder die Wirkstoff(e) in der Polymerschicht (3) nicht mit Wasser mischbar sind.

4. Transdermalsystem nach einem der vorstehenden Ansprüche, **dadurch** *gekennzeichnet,* **daß** das hydrophile Polymer Gelatine oder Celluloseester oder -ether oder Derivate davon umfaßt.

5. Transdermalsystem nach einem der vorstehenden Ansprüche, **dadurch** *gekennzeichnet,* **daß** die Polymerschicht (3) perforiert ist, so daß zumindest die Haftkleberschicht (4) mit auf der anderen Seite der Polymerschicht (3) gelegenen Schichten (1, 2) in Kontakt treten kann.

6. Transdermalsystem nach einem der vorstehenden Ansprüche, **dadurch** *gekennzeichnet,* **daß** die Wirkstoffe in den jeweiligen Schichten als immobilisierte Wirkstofflösung(en) oder -dispersion(en) vorliegen.

7. Transdermalsystem nach einem der Ansprüche 2 bis 6, **dadurch *gekennzeichnet*, daß**

    a) die Schichten (2) und (4) wirkstoffrei sind, oder
    b) die Schicht (2) wirkstoffhaltig und die Schicht (4) wirkstoffrei ist, oder
    c) die Schicht (2) wirkstoffrei und die Schicht (4) wirkstoffhaltig ist, oder
    d) die Schichten (2) und (4) wirkstoffhaltig sind.

8. Transdermalsystem nach einem der vorstehenden Ansprüche, **dadurch *gekennzeichnet*, daß** die Dicke der Schicht (4) 10 bis 300 μm, vorzugsweise 30 bis 100 μm beträgt.

9. Transdermalsystem nach einem der Ansprüche 2 bis 8, **dadurch *gekennzeichnet*, daß** die Dicke der Schicht (2) 1 bis 300 μm, vorzugsweise 3 bis 100 μm beträgt.

10. Verfahren zur Herstellung eines Transdermalsystems nach einem der vorstehenden Ansprüche, **dadurch *gekennzeichnet*, daß** in frei wählbarer Reihenfolge eine Haftschicht (4) auf eine Schutzschicht (5) aufgebracht wird, auf die Haftschicht (4) eine wirkstoffhaltige, gegebenenfalls Perforationen aufweisende Polymerschicht (3) aufgebracht wird, auf die Polymerschicht (3) gegebenenfalls eine weitere Haftschicht (2) aufgebracht und auf die oberste Schicht eine Abdeckschicht (1) aufgebracht wird.

11. Transdermalsystem nach einem der vorstehenden Ansprüche, **dadurch *gekennzeichnet*, daß** die Abdeckschicht (1, 11) ein oder mehrere wasserdampfundurchlässige(s) Material(ien), insbesondere Polyester, vorzugsweise Polyterephthalsäureester, oder Polypropylen oder Polyethylen, oder ein oder mehrere wasserdampfdurchlässige(s) Material (ien), insbesondere Polyurethan, oder ein oder mehrere Gewebe oder Vliese umfaßt.

12. Transdermalsystem nach einem der vorstehenden Ansprüche, **dadurch *gekennzeichnet*, daß** die Haftkleberschicht (4, 14) und/oder die Haftkleberschicht (2) unabhängig voneinander druckempfindliche Haftkleberschichten sind.

13. Transdermalsystem nach einem der vorstehenden Ansprüche, **dadurch *gekennzeichnet*, daß** die Haftkleberschicht (4, 14) und/oder die Haftkleberschicht (2) ein Netz, Vlies oder Gewebe enthalten, wobei deren Faden- oder Faserstärke vorzugsweise kleiner als die Haftkleberschichtdicke ist.

14. Transdermalsystem nach einem der vorstehenden Ansprüche, **dadurch *gekennzeichnet*, daß** die Schutzschicht (5, 15) wiederablösbar ist und insbesondere eine silikonisierte Kunststoffolie oder ein Silikonpapier ist.

15. Transdermalsystem nach einem der vorstehenden Ansprüche, **dadurch *gekennzeichnet*, daß** der Wirkstoff Testosteron, Nitroglycerin oder Gemische davon umfaßt.

16. Verwendung eines Transdermalsystems nach einem der vorstehenden Ansprüche zur Herstellung eines Arzneimittels zur Behandlung von Angina pectoris, zum Nicotinentzug oder bei Testosteronmangelerscheinungen.

**Claims**

1. A transdermal system comprising:

    a) a cover layer (1, 11),
    b) a water-soluble material that can be dissolved by moisture of the skin,
    c) an optionally active-ingredient-containing adhesive layer (4, 14), and
    d) a protective layer that is detachable therefrom,

    the transdermal system being **characterized by** an active-ingredient-containing polymer layer (3) in which the active ingredient is present in the form of a non-water-miscible active ingredient solution or of a non-water-miscible active ingredient dispersion in a water-soluble polymer.

2. A transdermal system according to claim 1, **characterized by** an adhesive layer (2) arranged between the active-ingredient-containing polymer layer (3) and the cover layer (1), which adhesive layer (2) optionally contains active ingredient.

3. A transdermal system according to one of the preceding claims, **characterized in that** the active ingredient(s) in the polymer layer (3) is (are) not miscible with water.

4. A transdermal system according to any one of the preceding claims, **characterized in that** the hydrophilic polymer comprises gelatin or cellulose esters or cellulose ethers or derivatives thereof.

5. A transdermal system according to any one of the preceding claims, **characterized in that** the polymer layer (3) is perforated, so that at least the adhesive layer (4) can come into contact with layers (1, 2) disposed on the other side of the polymer layer (3).

6. A transdermal system according to any one of the preceding claims, **characterized in that** the active ingredients are present in the relevant layers in the form of (an) immobilized active ingredient solution(s) or dispersion(s).

7. A transdermal system according to any one of claims 2 to 6, **characterized in that**

a) the layers (2) and (4) are free of active ingredient, or
b) the layer (2) contains active ingredient and the layer (4) is free of active ingredient, or
c) the layer (2) is free of active ingredient and the layer (4) contains active ingredient, or
d) the layers (2) and (4) contain active ingredient.

8. A transdermal system according to any one of the preceding claims, **characterized in that** the thickness of the layer (4) is from 10 to 300 µm, preferably from 30 to 100 µm.

9. A transdermal system according to any one of claims 2 to 8, **characterized in that** the thickness of the layer (2) is from 1 to 300 µm, preferably from 3 to 100 µm.

10. A process for the manufacture of a transdermal system according to any one of the preceding claims, **characterized in that**, in a freely selectable order, an adhesive layer (4) is applied to a protective layer (5), an active-ingredient-containing polymer layer (3) optionally having perforations is applied to the adhesive layer (4), a further adhesive layer (2) is optionally applied to the polymer layer (3), and a cover layer (1) is applied to the top layer.

11. A transdermal system according to any one of the preceding claims, **characterized in that** the cover layer (1, 11) comprises one or more water-vapour-impermeable material(s), especially polyester, preferably polyterephthalic acid ester, or polypropylene or polyethylene, or one or more water-vapour-permeable material(s), especially polyurethane, or one or more woven or non-woven fabrics.

12. A transdermal system according to any one of the preceding claims, **characterized in that** the adhesive layer (4, 14) and/or the adhesive layer (2) are, independently of each other, pressure-sensitive adhesive layers.

13. A transdermal system according to any one of the preceding claims, **characterized in that** the adhesive layer (4, 14) and/or the adhesive layer (2) contain a net, a non-woven fabric or a woven fabric, the thread or fibre thickness thereof preferably being less than the thickness of the adhesive layer.

14. A transdermal system according to any one of the preceding claims, **characterized in that** the protective layer (5, 15) is re-detachable and is especially a siliconised plastics film or a silicone paper.

15. A transdermal system according to any one of the preceding claims, **characterized in that** the active ingredient comprises testosterone, nitroglycerine or mixtures thereof.

16. The use of a transdermal system according to any one of the preceding claims for the production of a pharmaceutical preparation for the treatment of Angina pectoris, for nicotine withdrawal or in the case of testosterone deficiency symptoms.

**Revendications**

1. Système transdermique qui comprend :

(a) une couche de recouvrement (1, 11),
(b) une matière soluble dans l'eau, qui peut être dissoute par l'humidité de la peau,
(c) une couche d'adhésif (4, 14) contenant éventuellement un agent actif, et
(d) une couche de protection détachable de cette dernière,

ledit système transdermique étant **caractérisé par** la présence d'une couche de polymère (3), contenant un agent actif, couche dans laquelle l'agent actif est présent sous la forme d'une solution d'agent actif, non-miscible à l'eau, ou d'une dispersion d'agent actif, non-miscible à l'eau, dans un polymère soluble dans l'eau.

2. Système transdermique selon la revendication 1, **caractérisé par** la présence d'une couche d'adhésif (2), contenant éventuellement un agent actif, disposée entre la couche de polymère (3) contenant un agent actif et la couche de recouvrement (1).

3. Système transdermique selon la revendication 1 ou 2, **caractérisé en ce que** l'agent actif ou les agents actifs présents dans la couche de polymère (3) ne sont pas miscibles à l'eau.

4. Système transdermique selon l'une quelconque des revendications précédentes, **caractérisé en ce que** le polymère hydrophile comprend de la gélatine ou un ester ou éther cellulosique ou des dérivés de ces produits.

5. Système transdermique selon l'une quelconque des revendications précédentes, **caractérisé en ce que** la couche de polymère (3) est perforée, de sorte qu'au moins la couche d'adhésif (4) peut entrer en contact avec les couches (1, 2) placées de l'autre côté de la couche de polymère (3).

6. Système transdermique selon l'une quelconque des revendications précédentes, **caractérisé en ce que** les agents actifs sont présents dans les diverses couches sous la forme de solution(s) ou de dispersion(s) d'agents actifs immobilisés.

7. Système transdermique selon l'une quelconque des revendications 2 à 6, **caractérisé en ce que** :

> a) les couches (2) et (4) sont exemptes d'agents actifs, ou bien
> b)la couche (2) contient un agent actif et la couche (4) est exempte d'agents actifs, ou bien
> c)la couche (2) est exempte d'agents actifs et la couche (4) contient un agent actif, ou bien
> d)les couches (2) et (4) contiennent toutes les deux un agent actif.

8. Système transdermique selon l'une quelconque des revendications précédentes, **caractérisé en ce que** l'épaisseur de la couche (4) est de 10 à 300 µm, de préférence de 30 à 100 µm.

9. Système transdermique selon l'une quelconque des revendications 2 à 8, **caractérisé en ce que** l'épaisseur de la couche (2) est de 1 à 300 µm, de préférence de 3 à 100 µm.

10. Procédé de préparation d'un système transdermique selon l'une quelconque des revendications précédentes, **caractérisé en ce qu'**on réalise, dans un ordre choisi librement, les opérations suivantes : on applique une couche d'adhésif (4) sur une couche de protection (5), on applique sur la couche d'adhésif (4) une couche de polymère (3) contenant un agent actif et présentant éventuellement des perforations, on applique éventuellement sur la couche de polymère (3) une autre couche d'adhésif (2) et on applique sur la couche supérieure une couche de recouvrement (1).

11. Système transdermique selon l'une quelconque des revendications précédentes, **caractérisé en ce que** la couche de recouvrement (1, 11) comprend une ou plusieurs matières imperméables à la vapeur d'eau, en particulier du polyester, de préférence du poly(ester de l'acide téréphtalique), ou du polypropylène ou du polyéthylène, ou bien une ou plusieurs matières perméables à la vapeur d'eau, en particulier du polyuréthane, ou bien un ou plusieurs tissus ou nappes non-tissées.

12. Système transdermique selon l'une quelconque des revendications précédentes, **caractérisé en ce que** la couche d'adhésif (4, 14) et/ou la couche d'adhésif (2) sont des couches d'adhésif sensibles à la pression, indépendamment l'une de l'autre.

13. Système transdermique selon l'une quelconque des revendications précédentes, **caractérisé en ce que** la couche d'adhésif (4, 14) et/ou la couche d'adhésif (2) contiennent un filet, une nappe non-tissée ou un tissu dont les fils ou les fibres ont une épaisseur qui est de préférence inférieure à l'épaisseur de la couche d'adhésif.

14. Système transdermique selon l'une quelconque des revendications précédentes, **caractérisé en ce que** la couche de protection (5, 15) est redétachable et est constituée en particulier d'une feuille de matière plastique siliconée ou d'un papier siliconé.

15. 14-Système transdermique selon l'une quelconque des revendications précédentes, **caractérisé en ce que** l'agent actif comprend de la testostérone, de la nitroglycérine ou un mélange des deux.

16. Utilisation d'un système transdermique selon l'une quelconque des revendications précédentes, pour la préparation d'un médicament destiné au traitement de l'angine de poitrine, de la privation de nicotine ou des symptômes d'un manque de testostérone.

Verankerung

Abdeckschicht (1)
Haftkleberschicht (2)
Polymerschicht (3)
Haftkleberschicht (4)
Schutzschicht (5)

Abb. 1

**In vitro Hautpermeation Testosteron Beispiel 1 vs. Vergleichsbeispiel**

permeirte Menge in µg/cm²

Zeit (h)

♦ 2070
▼ 2070/Gelatine

EP 1 079 815 B1

In vitro Hautpermeation Testosteron Beispiel 2 vs. Vergleichsbeispiel